Europäisches Patentamt

European Patent Office

Office européen des brevets

(19)

(11) Veröffentlichungsnummer: **0 360 270**
**A2**

(12) **EUROPÄISCHE PATENTANMELDUNG**

(21) Anmeldenummer: **89117480.7**

(22) Anmeldetag: **21.09.89**

(51) Int. Cl.5: **A61F 7/02 , A61M 35/00**

(30) Priorität: **23.09.88 DE 3832451**

(43) Veröffentlichungstag der Anmeldung:
**28.03.90 Patentblatt 90/13**

(84) Benannte Vertragsstaaten:
**AT BE DE FR GB LU NL SE**

(71) Anmelder: **Toniolo, Beat A.**
**Stettemerstrasse 107**
**CH-8207 Schaffhausen(CH)**

(72) Erfinder: **Toniolo, Beat A.**
**Stettemerstrasse 107**
**CH-8207 Schaffhausen(CH)**

(74) Vertreter: **Herrmann-Trentepohl, Werner,**
**Dipl.-Ing. et al**
**Kirschner, Grosse, Bockhorni Forstenrieder**
**Allee 59**
**D-8000 München 71(DE)**

(54) **Therapie-Kompresse und Kissen hierfür.**

(57) Um auf einfache und hygienisch einwandfreie Weise Kompressionsverbände mit Therapiepflastern und Anwendung von Kälte zu kombinieren, weist die Kompresse eine elastische Bandage (1) auf, welche Haltetaschen (5, 6) für ein Wirkstoffkissen (3) und ein Kühlkissen (2) besitzt. Je nach Indikation kann wahlweise ein geeignetes Wirkstoffkissen verwendet werden.

Fig.1

## Therapie-Kompresse und Kissen hierfür

Die Erfindung betrifft eine Therapie-Kompresse sowie darin einsetzbare Wirkstoff- bzw. Kühlkissen gemäß dem Oberbegriff der Patentansprüche 1 bzw. 7.

Es ist bekannt, Entzündungen, Schwellungen und andere Läsionen an Körperteilen des Bewegungsapparates durch die Anwendung von Wirkstoffen (Salben), Kälte und Kompression zu therapieren. Hierzu sind denn auch Wirkstoff-Pflaster, Kältebeutel, Kompressen etc. vielfach bekannt. Allerdings sind bisher diese Therapien meist getrennt erfolgt und die Kombination mehrerer solcher Behandlungsformen war umständlich und materialintensiv. Therapiepflaster werden in der Regel nach Gebrauch weggeworfen und lassen sich auch nur schwer mit Kältebeuteln und/oder Kompressen sinnvoll kombinieren. Oft ist die Applikation für Laien schwierig und hygienisch bedenklich.

Hier will die vorliegende Erfindung Abhilfe schaffen. Es wird damit angestrebt, die erwähnten Therapieformen in sauberer, für den Laien einfach handhabbarer und beliebig kombinierbarer Weise anwendbar zu machen.

Erfindungsgemäß wird dies durch die Merkmale von Anspruch 1 erreicht, wobei zweckmäßige Weiterbildungen durch die in den weiteren Ansprüchen enthaltenen Merkmale gekennzeichnet sind.

Nach Maßgabe der Erfindung werden mittels einer einzigen Kompresse auf einfache Weise vielfache Anwendungen ermöglicht. Das Verbrauchsmaterial beschränkt sich auf die Wirkstoffkissen als solche, während alle übrigen Komponenten mehrfach wiederverwandt werden können.

Nachfolgend wird anhand der Zeichnungen ein bevorzugtes Ausführungsbeispiel der Erfindung näher beschrieben. Es zeigen:

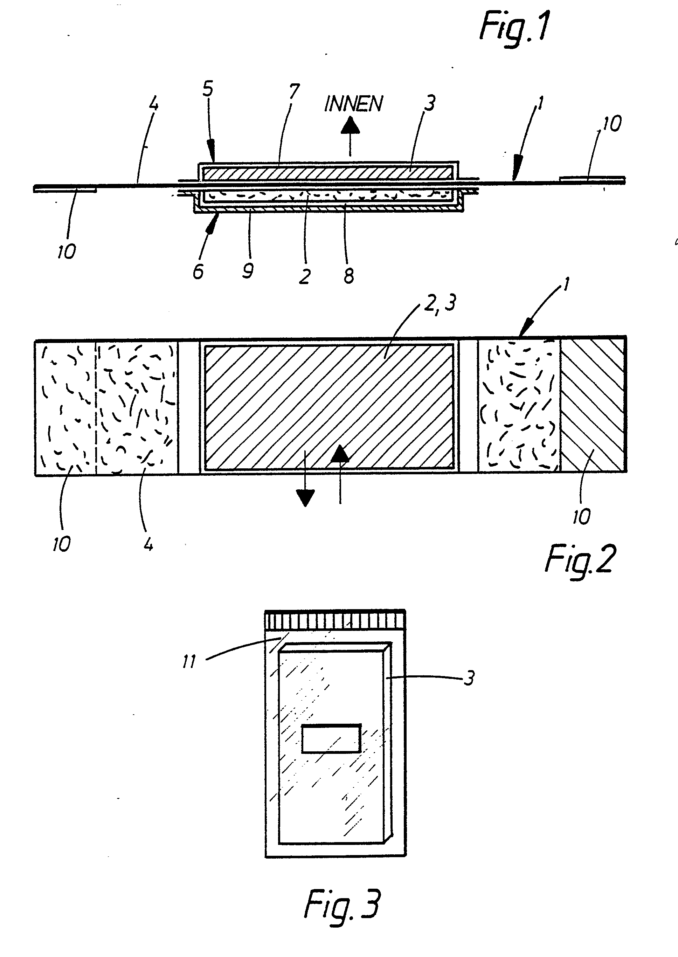

Fig. 1 die Therapiekompresse mit eingesetzten Wirkstoff- und Kühlkissen im Längsschnitt,

Fig. 2 dieselbe Kompresse in der Aufsicht, und

Fig. 3 ein Wirkstoffkissen in verpacktem Zustand.

Der grundsätzliche Aufbau der Kompresse besteht aus drei Hauptkomponenten, nämlich der Bandage 1, dem Kühlmittelkissen 2 und einem jeweils passend gewählten Wirkstoffkissen 3. Bis auf die letztgenannte Komponente sind alle Teile mehrfach wiederverwendbar. Die Bandage 12 besteht aus einem elastischen Textilband 4, an welchem im mittleren Bereich Haltetaschen 5, 6 für die Aufnahme des Kühlkissens 2 und des Wirkstoffkissens 3 angenäht sind. Die Haltetaschen sind nach einer Seite hin offen, so daß dort das Kühlmittel- und das Wirkstoffkissen eingeschoben bzw. entnommen werden kann. Die Tasche 6 für das Kühlkissen ist mit Vorteil an seiner Innenseite mit einer wasserundurchlässigen Folie oder Beschichtung 9 versehen, um ein Austreten von Kondensationsflüssigkeit zu vermeiden. Die Tasche 5 für das Wirkstoffkissen ist dagegen nach der Körperseite hin mit einem für die Wirkstoffe durchlässigen Gewebe, z.B. einem Gazegewebe 7 versehen. Diese Tasche ist an der kör perseitigen Bandagefläche angeordnet, wogegen die Tasche 6 für das Kühlkissen auf der Außenseite liegt. Die Kühlung erfolgt damit durch das Wirkstoffkissen hindurch.

Zur Kälteisolation besitzt die Tasche 6 für das Kühlkissen zweckmäßigerweise an der Außenseite eine Isolationsschicht 8, um die Kälteverluste an die Umgebung herabzusetzen. An den Enden weist die Bandage 1 eine geeignete Befestigungseinrichtung 10 auf, zweckmäßigerweise in Form eines Klettverschlusses, mittels welcher die Bandage unter Zug fixiert werden kann.

Das Kühlkissen 3 enthält eine gelartige, verformbare, in einem flexiblen Beutel eingeschlossene Kühlmasse an sich bekannter Art. Zur Aktivierung ist das Kissen während einer gewissen Zeit in einen Kühlschrank zu legen. Bei Anwendungen im Freien können aktivierte Kühlkissen in wärmeisolierten Behältern mitgeführt werden. Das Kühlkissen ist beliebig oft wiederverwendbar.

Das Wirkstoffkissen 3 ist bereits herstellerseitig mit den Wirkstoffen versehen, die insbesondere antiphlogistische, schmerzlindernde und wundheilende Bestandteile haben können, welche durch die Haut aufgenommen werden und je nach Applikation in unterschiedlichen Trägerstoffen gelöst sein können. Als Beispiel einer Wirkstoffkombination sei erwähnt:

2% Ol.Menthae: kühlend bei frischen od. chron. Muskel-und Gelenksverletzungen, bakterizid, analgetisch, epitelisierend,

4% Symphytum off.:schmerzlindernd, adstringierend, antiphlogistisch, fördert regenerative Vorgänge,

2% Ol.Hyperi.: antiphlogistisch, adstringierend, wundheilend,

1% Ol.Arnicae: resorptionsfördernd, analgetisch, wundheilungsfördernd,

1%/0,5%Indometacin:antiphlogistisch, analgetisch, wirksame Prostaglandinsynthesehemmung, gute Penetration durch Haut, Lokalwirkung

Excip. ad. 100%: Trägerstoffe - Öl-Grundlage, W/O-Emulgatoren.

Die Wirkstoffkissen 3 werden in hermetisch verschlossenen Aufreißpackungen 11 aufbewahrt und mitgeführt. Zum Gebrauch wird das gewünschte Wirkstoffkissen durch Aufreißen der Packung 11 entnommen und in die entsprechende Haltetasche 5 eingeschoben. In die andere Haltetasche 6 an der Bandagenaußenseite wird bei Bedarf ein Kühlkissen eingesetzt. Beide zusammen bilden eine Art flexibles Pflaster an der Bandage 1 und passen sich unter dem Zug der Bandage an die Körperform an.

Der Anwendungsbereich der beschriebenen Kompresse reicht je nach verwendeter Wirkstoffkombination von chronischen Erkrankungen des Bewegungsapparats (Rheuma, Arthrose, Arthritis, Gicht) über Sportverletzungen bis zu Venen- und Krampfaderner krankungen. Die Kompresse kann bedarfsweise auch als reines Kältepflaster, Wirkstoffpflaster oder Druckverband eingesetzt werden. Sie ist sogleich an die jeweilige Diagnose anpaßbar und bietet eine saubere und hygienisch einwandfreie Applikationsform ohne Salben, Umschläge etc. Mit der Kompresse kann eine langanhaltende Wirkstoffabgabe erzielt werden. Die Kühlmittelkissen können unabhängig vom Wirkstoffkissen mehrfach ausgewechselt werden, um dem Kühleffekt über längere Zeit sicherzustellen.

## Ansprüche

1. Therapie-Kompresse mit einer Bandage zur Umschlingung eines Körperteiles, insbesondere Arm oder Beim, die im Endbereich mit einer Befestigungseinrichtung zur Fixierung versehen ist, dadurch **gekennzeichnet,** daß zwischen den Enden mindestens eine Haltetasche (5, 6) für die Aufnahme eines Kissens (2, 3) mit Wirkstoffen oder Kühlmittel vorgesehen ist.

2. Kompresse nach Anspruch 1, dadurch **gekennzeichnet,** daß die Bandage (4) mit einem Klebstoff zwecks direkter Befestigung auf dem zu behandelnden Gewebe beschichtet ist.

3. Kompresse nach Anspruch 1 oder 2, dadurch **gekennzeichnet,** daß zwei übereinanderliegende Haltetaschen (5, 6) vorgesehen sind, wobei die eine Haltetasche (6) zur Aufnahme eines Kühlkissens (2) und die andere Haltetasche (5) zur Aufnahme eines Wirkstoff enthaltenden Kissens (3) ausgebildet ist.

4. Kompresse nach Anspruch 3, dadurch **gekennzeichnet,** daß die Haltetasche (6) für das Kühlkissen (2) aus wasserundurchlässigem Material gebildet ist.

5. Kompresse nach einem der Ansprüche 3 und 4, dadurch **gekennzeichnet,** daß die Haltetasche für das Wirkstoffkissen (3) ein körperseitig für den Wirkstoff durchlässiges Gewebe aufweist.

6. Kompresse nach einem der vorangehenden Ansprüche, dadurch **gekennzeichnet,** daß die Bandage (1) elastisch und die Befestigungseinrichtung (10) zugfest ausgebildet ist.

7. Kissen für Therapie-Kompresse nach einem der vorangehenden Ansprüche, dadurch **gekennzeichnet,** daß es eine gelartige, verformbare, Kühlmasse aufweist.

8. Kissen nach Anspruch 7, dadurch **gekennzeichnet,** daß es als Beutel aus vorzugsweise Kunststoffolie oder anderem geeignetem Material ausgebildet ist, in welchen die Kühlmasse einführbar ist und durch Verschweißen, Verkleben oder auf eine andere geeignete Weise verschließbar ist.

9. Kissen für Therapie-Kompresse nach einem der Ansprüche 1 bis 6, dadurch **gekennzeichnet,** daß es einen mit Wirkstoffen getränkten Träger beinhaltet, welcher im in der Therapie-Kompresse eingesetzten Zustand diesen an den zu behandelnen Körperteil abgibt.

10. Kissen nach Anspruch 9, dadurch **gekennzeichnet,** daß es aus einem für den Wirkstoff durchlässigen Material besteht und biz zur Anwendung zusätzlich dicht verpackt ist mit einer für den Wirkstoff undurchlässigen Hülle (11).

*Fig.1*

INNEN

*Fig.2*

*Fig.3*